# EUROPEAN PATENT APPLICATION

(11) **EP 2 400 314 A1**
(43) Date of publication of application: **28.12.2011**
(21) Application number: 10006134.0
(22) Date of filing: 14.06.2010
(51) Int. Cl.: G01R 33/3815, H01F 6/00, F17C 13/08, G01R 33/38, G01R 33/48

(54) **Superconducting magnet arrangement and method of mounting thereof**

(71) Applicant: Agilent Technologies U.K. Limited, IQ Winnersh Wokingham Berkshire RG41 5TP (GB)
(72) Inventor: Warner, Rory John, Oxford OX3 0DU (GB); Courtney, Alistair, G., Oxford OX2 9LF (GB); Haynes, Nigel, Northhamptonshire NN15 (GB)
(74) Representative: Zimmermann, Tankred Klaus

(57) **Abstract**

The method provides a superconducting magnet arrangement (10), a method of mounting the magnet arrangement, and a kit for assembling a magnet arrangement. The magnet arrangement comprises: a pair of spaced apart coil members (34a, 34a', 34a"; 34b, 34b', 34b"), wherein the coil members are axially aligned along a common axis (12); a pair of spaced apart preferably toroidal chambers (33a, 33b), wherein the chambers are aligned along the common axis (12), wherein each chamber houses one of the coil members (34a, 34a', 34a"; 34b, 34b', 34b") and wherein each chamber is adapted to receive and store a liquid coolant; and a plurality of support structures (17, 35a, 35b) arranged about a periphery of the chambers (33a, 33b) and mechanically coupled therewith providing a predetermined gap between said chambers and fixing the position of said superconducting coil members (34a, 34a', 34a"; 34b, 34b', 34b").

## Description

### FIELD OF THE INVENTION

The present invention relates generally to a superconducting magnetic apparatus suitable for Magnetic Resonance Imaging (MRI) system compatible with a radiation source for simultaneously delivering the radiation to the subject. More particularly, the invention relates to superconducting magnet for MRI magnet apparatus for generating magnetic field suitable for magnetic resonance imaging and having an open access for providing radiation of a subject.

### BACKGROUND OF THE INVENTION

The superconducting MRI magnet apparatus generating a strong uniform magnetic field for magnetic resonance imaging, and providing a structure compatible with simultaneous radiation generally provides the magnetic field by a pair of split apart coils with the subject positioning along the common axis of the coils, when the radiation is entering between the coil pair perpendicular to the common axis of the coil pair.

A conventional system whereby the radiation is produced by a linear accelerator mounted on a gantry that rotates about the common axis of the two coils permitting radiation to be deposited in the open structure between the coil pair is described in the US Patents 6,198,957 B1 and 6,366.798 B2. The radiation is incident on the subject perpendicular to the axis of the coils and incident from various angles around the subject. The disclosure of the US 6,198,957 only mentioning that in the combination of the radio-radiation machine and MRI system, the DC magnetic field for the magnetic resonance imaging system may be produced by a superconducting magnet.

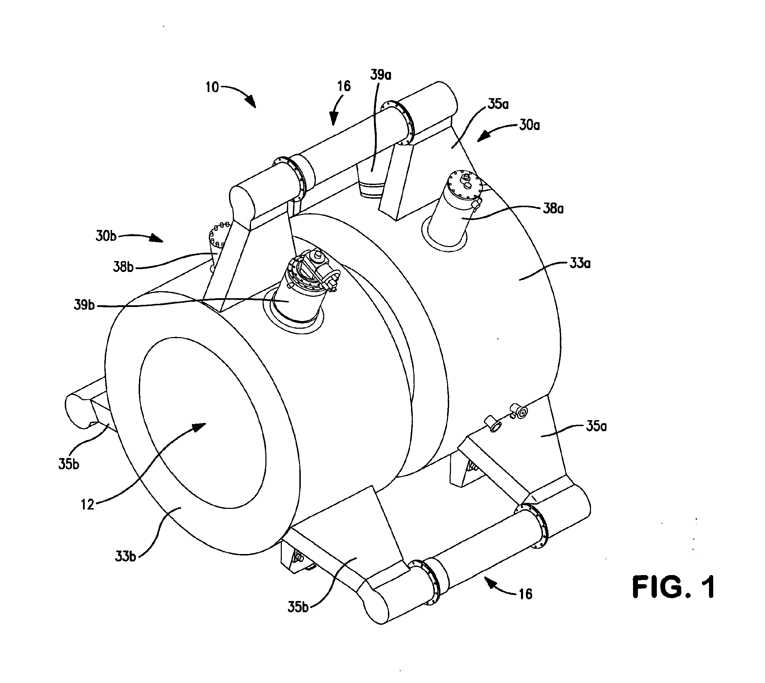

Another system for delivering radiation while simultaneously imaging soft tissue by MRI is described in US Patent Publication No. US 2010/0113911. The main magnet of the MRI is a Helmholtz coil pair designed as a split solenoid with the subject couch running through the bore of the coil pair. The isotope source, typically cobalt-60, with a multi-leaf collimator is located in the gap between the coil pair, and is rotated axially around the subject couch. The drawings, Figs. 1- 4, show the coil pair mounted on a common base along with the gantry supporting the isotope source and multi-leaf collimator. In the references cited above the magnet coils are supported on a single base that is not temperature controlled. The coils would undergo flexure as current is applied or changed, causing changes in the magnetic field strength and uniformity making the system impractical except for non-superconducting magnets operated at very low magnetic field strengths.

The US Patent 5,414,399 describes a superconducting MRI magnet producing a vertically directed magnetic field, where the subject is positioned horizontally in the plane between an upper magnet coil and a lower magnet coil. Each magnet coil is contained within separate cryostat vessel, cooled by liquid Helium. Each coil with its cryostat is mounted on an end plate with the subject lying between coils and the supporting end plates. The two end plates are separated from each other by four ferromagnetic posts that provide return magnetic flux and fix the position of the coils. This arrangement gives access only to the side of a subject not providing perpendicular access for all angles around the subject for a radiation beam.

US Patent 5,939,962 teaches similar coil geometry as US patent '399, with a superconducting coil above and below the subject producing a vertically directed magnetic field. Each coil is mounted within its own cryostat; however no arrangement is made for supporting the cryostats. The coil geometry also does not provide access from all angles around the subject for a radiation beam. A sectional view of the conventional magnetic field generating apparatus shown in figure 6 appears to have a port along the magnetic filed axis that could be used for a radio-radiation beam along the direction of the magnetic field; however this arrangement still would not permit access to the subject from all angles. There is no teaching which would suggest how to arrange the two coils so that a radiation beam could be provided from all angles around the subject.

Another superconducting coil magnet system is described in EP 0 676 647, where a first coil assembly is located in a toroidal-shaped first coil housing surrounding a first bore having a longitudinal first axis. A second coil assembly is located in a toroidal-shaped second coil housing surrounding a second bore having a longitudinal second axis generally aligned with the first axis. The first and second housing form a vacuum enclosure. The two housings are coupled together with several hollow structural posts providing a single vacuum system. The structural posts are located between the two toroidal-shaped coil housing members thereby forming obstructions to radiation emanating from a source at a greater radial distance than the posts from the common axis of the two coil assemblies. The single vacuum system with assembly of coils forming a single large and heavy MRI magnet makes delivery to facilities with restricted access difficult.

In the attempt to achieve better access of the radiation beam to the subject it was proposed to mount a 6 MV accelerator on a gantry surrounding the exterior of a 1.5 T MRI system, enabling the accelerator to move in a circular arc about the axis of the magnetic field axis of the MRI.(B. W. Raaymakers et al, Phys. Med. Biol. Vol. 54, 2009, pgs N229-N237, "Integrating a 11.5 T MRI scanner with a 6 MV accelerator: proof of concept") The MRI magnet coils were disposed in a single cryostat so the radiation beam must pass through the walls of the cryostat thereby causing a weakening and scattering of the radiation beam before interacting with the subject being treated.

In view of the foregoing, there is a need for providing a superconducting magnet arrangement (which may be used for magnetic resonance imaging) that provides space for a radiation source and the radiation beam so that it can be directed perpendicular the subject and through a wide range of angles around a common axis. Alternatively or additionally there is need for a magnet arrangement kit or a method for simple'place of destination' assembling.

### SUMMARY OF THE INVENTION

To address the foregoing problems, in whole or in part, and/or other problems that may have been observed by persons skilled in the art, the present disclosure provides an apparatus as described by way of example in implementations set forth below.

The invention is defined in claims 1,8 and 15 respectively. Particular embodiments are set out in the dependent claims.

According to the preferred embodiment, separate cryostats are used to contain at least two coils for producing the magnetic field. Each cryostat has a preferably toroidal chamber that contains a superconducting coil member (having at least one field coil) and space for a coolant such as liquid Helium. The two cryostats may be identical or essentially identical in construction and face each other when assembled into a magnet. The two cryostats are supported a predetermined distance apart by a plurality of support structures mechanically attached to each cryostat. The support structure provides a mechanical link between the cryostat chambers (housing the coil members) at a radial distance from the center of the coil member for creating an unobstructed region between the coil members and that extends around the cryostat housing. The radial height and the separation distance between the two cryostats depend upon the requirements of e.g. a radio radiation system. Preferably the region is sufficiently large to accommodate a radiation source with no obstructions between it and the subject that would absorb or scatter the radiation beam.

In a preferred embodiment of the present invention the external supporting structure that fixes the relative positions of the MRI coils is provided with cooling means to maintain it at a constant temperature. This minimizes or eliminates any effect of room temperature variations that could cause expansion or contraction of the supporting structure causing variations in the magnetic field.

In an embodiment the two cryostats are connected and spaced apart with a predefined gap between the cryostats by connecting the cryostat chambers (which are preferably circular, ring-shaped or toroidal chambers) by two, three, four or more support structures. Each support structure supports the cryostat chambers in a predefined spatial relation to each other. Thereby the superconducting coil members arranged in the cryostat chambers are aligned to each other such that their (magnetic) axes of symmetry are collinear forming a common axis.

Due to mechanical stability it is favorable when the support structures are spaced around the periphery of the two cryostats with equal angle increments between the support structures, preferably with respect to the azimuthal distribution of the support structures around the common axis. Preferably the plurality of support structures comprises three support structures and the two support structures are spaced at equal angle increments about the common axis.

Preferably each one of the support structures extends beyond the outer dimensions of the cryostat chambers (the outer dimensions with respect to the common axis of the cryostat chambers). With respect to the common axis, the support structures extend radially or at least with a radial component of extension beyond the outer dimensions of the cryostat chambers. An axial component of each one of the support structures bridges the axial distance between the cryostat chambers. Preferably the axial component is completely or partially formed by a support spacer having an axial extension with respect to the common axis. The axial distance between the cryostat chambers results in a gap between the two facing outer side faces of the cryostat. In particular when the cryostat chambers are enclosed by a thermal shield and a vacuum case the gap is formed between the vacuum case side walls of the facing cryostat chambers.

The cryostat chambers are effective for housing the superconducting coil members and for storing liquid coolant for cooling the coil members to be superconducting. Preferably each cryostat chamber is enclosed by at least one thermal shield and a vacuum case. Preferably the support structure is enclosed by at least one thermal shield and a vacuum case. After mounting the support structure at both ends to the cryostat chambers, preferably each one of the at least one thermal shields of the cryostat chambers are mechanically and functionally coupled to the respective one of the at least one thermal shields of the support structures and/or the vacuum case of the cryostat chambers are mechanically and functionally coupled to the vacuum case of the support structures. Preferably the at least one thermal shield and the vacuum case enclose the cryostat chamber and the support structures like onion skins, wherein each cryostat chamber and each support structure is enclosed individually except the respective connection portions between each one of the support structures and each one of the cryostat chambers. This means that one common vacuum chamber is formed by the vacuum case around all cryostat chambers and all support structures such that preferably only one vacuum port for evacuation is required.

Preferably each coil member comprises one, two, three or more coil windings, wherein at least a portion or all of the coil windings are formed of superconducting material. Preferably the coil windings are arranged spaced from each other within the cryostat chamber. Preferably the winding plane of the one or more coil windings of each coil member is perpendicular or essentially perpendicular to the common axis.

According to a preferred embodiment the support spacer of each support structure is formed by a support tube, support bar or a plurality of axially extending rods or tubes. Preferably the support spacer is a mechanically rigid and lightweight element. The support spacer may have a flange at one or two axial ends thereof (axial ends when seen in the mounted state of the arrangement and with respect to the common axis forming the longitudinal axis of the support spacer), and respectively one or each one of the support pillars of the support structure has a correspondingly mating flange, mating with the flange of the support spacer. The flanges may be bolt and/or thread circles that are bolted together or flanges may be connected in any other way. Thus the 'on-site' mounting and adjustment of the magnet arrangement can be executed quickly.

In an embodiment a thermal shield sleeve for thermally shielding the support bar at its axial circumference has a flange at one or both axial ends of the thermal shield sleeve and one or both of the thermal shields of the support pillars have correspondingly mating flanges. So the flange(s) of the thermal shield and the thermal shield sleeve may be quickly connected on-site. In a further or an alternative embodiment a vacuum cover sleeve for enclosing the shield sleeve in a vacuum-tight manner is enclosing the thermal shield sleeve at its axial circumference and has a flange at one or both axial ends of the vacuum cover sleeve and one or both of the vacuum cases of the support pillars have correspondingly mating flanges. So the flange(s) of the vacuum cover sleeve and the vacuum cover may be quickly connected on-site. Preferably the length of the thermal shield sleeve and/or the vacuum cover sleeve may be varied, for example the thermal shield sleeve and/or the vacuum cover sleeve are formed telescopically expandable. In this case the vacuum cover sleeve may be formed of at least two sleeve elements axially slidable to each other, wherein in particular a vacuum sealing is provided between each two of the sleeve elements.

In an embodiment the support or suspension of the magnet arrangement is provided via at least one, preferably two of the support structures, e.g. two support structures are resting on a building floor. Thereby a 'heat bridge' between the cryostat chambers and the lab surroundings is provided distant from the vicinity of the cryostat chambers. Preferably the suspension or support points are arranged at the support pillars where locally reinforced thermal shield sections and vacuum case sections are provided for mechanical support of the respective support pillars while maintaining thermal shielding and vacuum enclosure.

In an embodiment of the invention a superconducting magnet for Magnetic Resonance Imaging apparatus comprises: a pair of spaced apart and axially aligned along a common axis superconducting coils: a pair of spaced apart toroidal chambers aligned along the common axis, each housing at least one of the superconducting coils and containing a liquid coolant; a plurality of support structures arranged about a periphery of the toroidal chambers and mechanically coupled therewith providing a predetermined gap between said toroidal chambers and fixing the position of said superconducting coils.

In an embodiment of the invention's method of forming of a superconducting magnet for MRI, the method comprises the steps of: orienting and coupling two cryostats, each with a toroidal chamber, via three spacer tube assemblies, each with a support tube and coupling means; positioning within each said toroidal chamber one or more spaced apart solenoidal superconducting magnetic field coils having a common axis; feeding a liquid coolant via a coolant inlet port coupled to respective toroidal chamber; coupling mechanically and thermally the support tubes of the three spacer tube assemblies by the coupling means to three support pillars extending outwardly from an outer surface of the toroidal chambers; placing a thermal shield sleeve of adjustable length around the support tube of each spacer tube assemble and each toroidal chamber and attached support pillars; positioning a vacuum case having a cover sleeve of adjustable length surrounding each said thermal shield; coupling said vacuum case with each said spacer tuber assembles; and adjusting length of the thermal shield sleeve and length of the cover sleeve.

Another advantage of the present invention is that the two cryostats, each with a field coil may be shipped as separate units with final attachment made at their destination. This permits easy movement of the two cryostats allowing for easy entrance to facilities with, for example, narrows doorways or otherwise limited access. The complete system can be assembled at their destination with relative ease, not requiring any welding, brazing or similar operations.

Providing a direct mechanical bridge between the two cryostat chambers via the support structures increases the mechanical stability of the arrangement, in particular when the coil members are powered. The mechanical suspension of the at least one thermal shield and the vacuum case may be designed mechanical 'weak' as compared to the mechanical rigidity between the cryostat chambers provided by the support structures. Symmetrical arrangement of the support structures around the cryostat chambers results in symmetrical displacements with respect to the common axis in case of thermal drifts. The radial extension of the support pillars with respect to the common axis provides an all-360°-around space free of barriers around the gap and in the gap between the cryostat chambers. The modularity of the system simplifies a change of the gap width between the cryostats by simply adjusting the axial length of the support spacer of each support structure (and preferably the length of the vacuum cover sleeve and/or thermal shield sleeve - if not readily provided by the variability of their axial length anyway). The change in gap width provides flexibility of the manufacturer as well of the user responding to changing demands. Also a change in the radial extension of the support structures can be solved by providing support pillars with different radial extension (with respect to the common axis). Thus adaptation to a change in radiation equipment that requires the space of the gap and the space radially extending beyond the gap is easily solved. Also the modularity of the magnet arrangement simplifies transport of the (partially) disassembled components of the arrangement to the site of final destination and mounting there - weight and/or dimensions of the components can be considered separately all the way from the factory to final destination.

The features of the superconducting magnet arrangement can be combined with the method of the invention or the magnet arrangement kit individually or in any arbitrary combination. The features of the method of the invention can be combined with the superconducting magnet arrangement or the magnet arrangement kit individually or in any arbitrary combination. The steps of the method may have a partially deviating order - in particular in view of the order of steps for assembling the magnet arrangement.

Other features and advantages of the invention will be or will become apparent to one with skill in the art upon examination of the following figures and detailed description.

### BRIEF DESCRIPTION OF THE DRAWINGS

The invention can be better understood by referring to the following figures. The components in the figures are not necessarily to scale. In the figures like reference numerals designate corresponding parts from different views.

Fig. 1 is a perspective view of the superconducting magnet for MRI magnetic apparatus formed by two spaced apart cryostats incorporating features of the invention.

Fig. 2 is a side view of the superconducting magnet of Fig. 1.

Fig 3 is a cross section showing one half of the superconducting magnet arrangement taken along the direction of the common axis of the magnetic field coils extending from the common axis through the remaining part of the apparatus.

Fig. 4A is a perspective view of the spacer tube assembly that determines the spacing between the two cryostats.

Fig. 4B is a cross sectional view of the spacer tube assembly of Fig. 4A.

### DESCRIPTION OF THE PREFERRED EMBODIMENTS

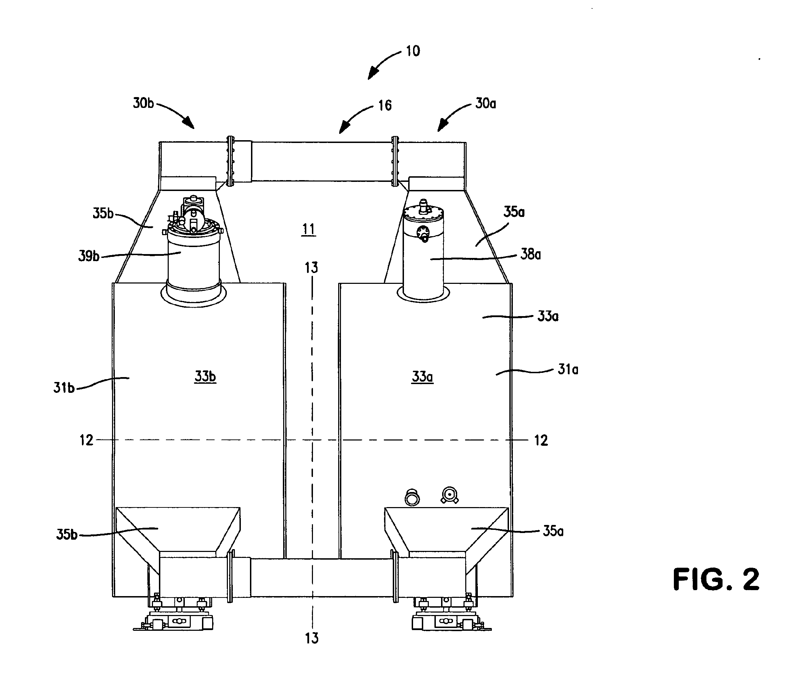

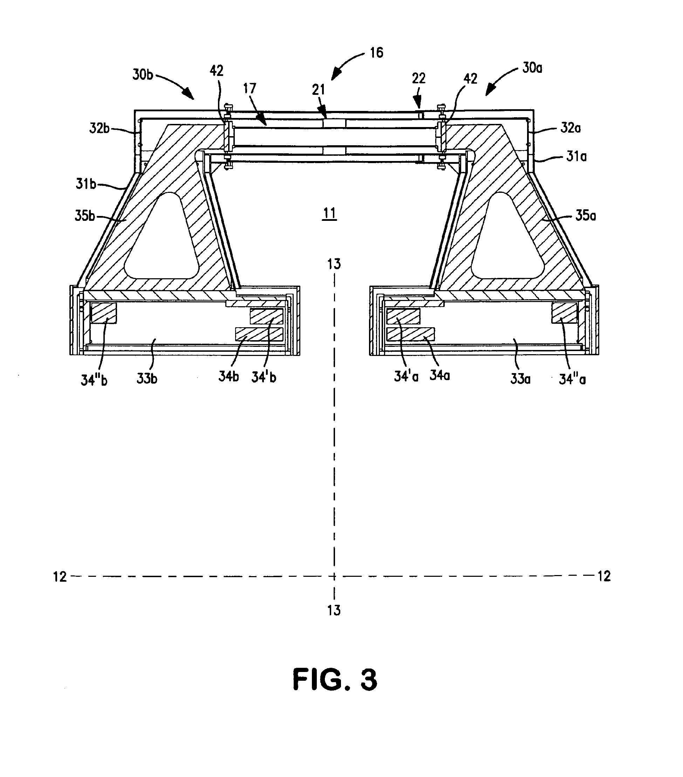

Figures 1 and 2 show the superconducting magnet **10** for magnetic resonance imaging (MRI) apparatus and its side view respectively in accordance with the present invention. The two inner side faces of the cryostats, which may be identical, face each other when coupled together. The superconducting magnet 10 is formed by a first cryostat **30a** with outer vacuum case **31a** and a second cryostat **30b** with outer vacuum case **31b**, which are mechanically coupled by three spacer tube assemblies **16** leaving an open space 11 between and around the opening between the two cryostats. Ports **38a** and **38b** are provided for charging cryostats **30a** and **30b** respectively with coolant, typically liquid Helium. Cryogenic coolers **39a** and **39b** cool the internal thermal shields **32a** and **32b** shown in figure 3. The magnet field coils within the cryostats **30a** and **30b** have their axes aligned along common axis **12.**

Figure 2 gives a better view of the open space **11** between support pillars **35a** and **35b**, and in the gap between the cryostats **30a** and **30b**. Plane **13** is perpendicular to common axis **12** and midway in the gap between the cryostats **30a** and **30b**.

Figure 3 is a cross sectional view of the superconducting magnet taken along the common axis **12** of the magnetic field coils, showing only the uppermost support structure above the common axis **12** including a cross section of support pillars **35a** and **35b** and spacer tube assembly **16**.

The cryostats **30a** and **30b** contain respective toroidal chambers **33a** and **33b** containing respective superconducting magnetic field coils **34a** and **34b** therein and the coolant (not shown). Depending upon the size of the MRI apparatus, the gap and the magnetic field uniformity requirement, one or more field coil windings may be required in each chamber. Figure 3 is illustrated with three field coil windings, **34a, 34a', 34a"** and **34**b, **34b', 34b"**, in each respective cryostat **30a** and **30b**. The number of field coil windings depends upon the size and spacing of the cryostats, and the required magnetic field uniformity. All windings of each cryostat may be connected in series there between and in series with persistence switch (not shown), or each may be connected to a separate persistence switch, with the charging leads and leads operating the persistence switch extending through each cryostat to charging apparatus (not shown) external to the cryostats. Each cryostat may also contain gradient coils and persistent shim coils with their leads extending through the cryostat wall to external power sources (not shown).

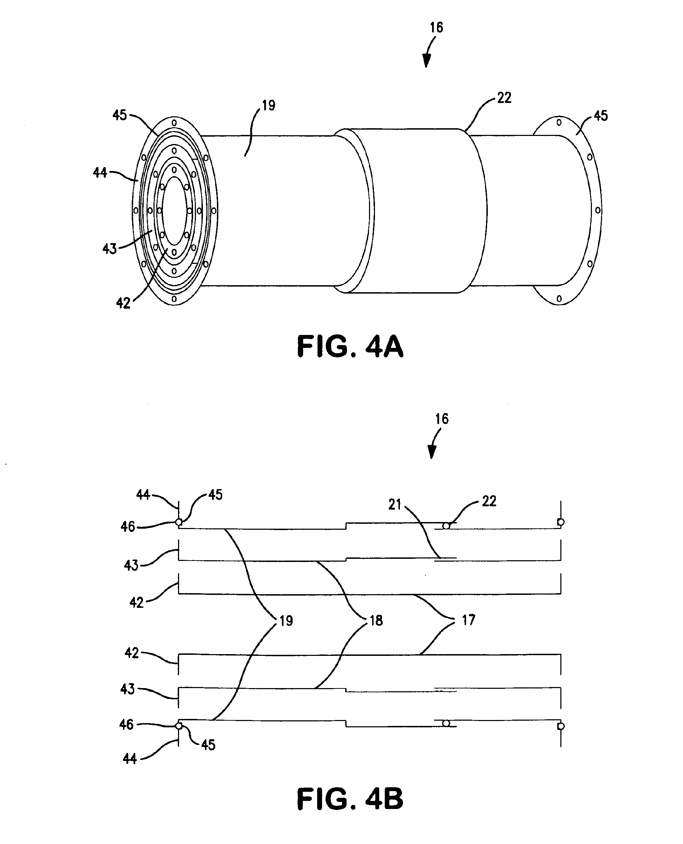

The toroidal chambers **33a** and **33b** and the axis of each coil **34a**, **34a', 34a"** and **34b**, **34b', 34b"** are aligned with the common axis **12**. Three support pillars **35a**, **35b** form part of the support system to provide the open space **11** between the cryostats. The base of three support pillars **35a, 35b** are fixed to the outer surface of the respective toroidal chamber **33a, 33b** and are spaced symmetrically around the toroidal chamber outer surface. The top ends of the support pillars have a bolt circle that matches the inner bolt circle **42** of the spacer tube assembly **16**, providing mechanical coupling between the cryostats (Figs. 4A. and 4B).

The superconducting magnet **10** is designed to provide a clear space **11** between and around the outside surface of the toroidal chambers **33a, 33b**. The described arrangement allows for enough space for a radio-radiation system to be installed therein, in close proximity to the subject.

An outer vacuum case **31a, 31b** surrounds the entire structure forming the outermost surface of each cryostat **30a, 30b**. The space between the outer vacuum case **31a**, **31b** and the toroidal chambers **33a,** 33b and the support pillars **35a, 35b** contains a thermal shield **32a**, **32b** (except where the support pillars are attached to the toroidal chambers.) This space is also pumped to a low pressure to provide good thermal insulation. The thermal shield **32a, 32b** is thermally coupled to the respective cryogenic cooler **39a, 39b**. Each cryostat **30a, 30b** has one cryogenic cooler **39a**, **39b** fixed to it to maintain its thermal shied at a low temperature.

Fig. 4A is a perspective view of a spacer tube assembly **16**, and Fig. 4B is a cross sectional view of a spacer tube assembly. One end of spacer tube assembly is mechanically coupled to the cryostat **30a** and the other end to cryostat **30b**. Each spacer tube assembly **16** has a central support tube **17** that provides the mechanical strength to oppose the large magnetic forces between field coils **34a** and coil **34b**. A bolt circle **42** at each end of the support tube allows it to be mechanically attached and detached to a matching bolt circles fixed to the support pillars **35a** and **35b** on cryostats **30a** and **30b** respectively.

The support tube **17** is surrounded by a thermal shield sleeve **18** that is surrounded by an outer vacuum cover sleeve **19**. The thermal shield sleeve **18** has a sliding joint **21** enabling the length to be shortened during magnet assembly to be less then the length of support tube **17**. This sliding joint does not need to be vacuum-tight because during operation the same vacuum pressure is maintained on both sides of the sliding joint. A bolt circle **43** fixed to each end of the thermal shield sleeve allows it to be mechanically and thermally coupled to matching bolt circles fixed to the thermal shields **32a** and **32b** of the cryostats **30a** and **30b**.

The outer vacuum cover sleeve **19** has a sliding O-ring seal **22** enabling its length to also be shortened during assembly. The O-ring seal **22** makes this sliding joint vacuum tight. The vacuum cover sleeve **19** with its bolt circles **44** at each end permit it to be mechanically coupled to a matching bolt circle fixed to the outer vacuum case **31a, 31b** of cryostats **30a** and **30b**. Bolt circles **44** contain an O-ring seal **46** in groove **45** forming vacuum-tight seals when coupled to the corresponding bolt circle fixed to the outer vacuum case of the cryostats.

The central support tube **17** is cooled by thermal contact with the support pillars on each side, which in turn are cooled by their thermal contact with the coolant in the respective toroidal chamber. The thermal shield sleeve 1**8** is cooled by thermal coupling to the respective thermal shields of each cryostat which in turn are cooled by cryogenic coolers **39a and 39b** fixed to cryostats **30a** and **30b** respectively. The outer vacuum cover sleeve **19** is coupled to the outer vacuum cases **31a** and **31b** of the cryostats **30a** and **30b** by vacuum tight seals. The thermal insulation provided by these structures greatly reduces or eliminates the effect of room temperature variations that could cause expansion or contraction of the magnet structure that in turn could cause variations in the strength and uniformity of the magnetic field.

The spacer tube assembly is made so that it can be readily attached and detached using just a wrench to screw and unscrew the bolts used fix the bolt circles on the ends of the spacer tube assembly with the corresponding bolt circles on the cryostats. This allows for easy movement of the two cryostats to facilities with narrow doorways or otherwise limited access. The final assembly can then take place in the facility at the magnet destination. Attaching two cryostats together to form a complete magnet involves bolting of the inner bolt circle of support tubes **17** of three spacer tube assemblies **16** to cryostat **30a;** then slipping the three thermal shield sleeves **18** over the three attached support tubes and bolt central bolt circle them to cryostat **30a;** fixing the O-ring seals **46** in the O-ring grooves **45** of outer bolt circles **44**, and slipping the three outer vacuum cover sleeves **19** over the three thermal shield sleeves and bolt them to cryostat **30a.**

The thermal shield sleeves **18** have a sliding joint **21** and the outer vacuum cover sleeves **19** have a sliding O-ring seal **22**. Shorten the effective length of both by pushing on the exposed ends. This exposes the protruding bolt circle **42** of the support tubes **17**. Align the two cryostats, making sure the O-ring seals **46** are seated in grooves **45** and bolt the three support tubes **17** to the support pillars **35b**. The thermal shield sleeves **18** slide out to match their bolt circles **43** with the corresponding bolt circles fixed to the thermal shields of cryostat **30b**. They are attached together by bolting circle **43** to the corresponding bolt circle on thermal shield **32b**. The remaining outer vacuum cover sleeves **18** slides out to match bolt circles **44** with the corresponding bolt circle on the cryostat. With the O-ring seal **46** in groove **45**, the bolt circles **44** of outer vacuum cover sleeves **19** are bolted to the corresponding bolt circles on outer vacuum case **31b**. Toroidal chambers serve as vessels for the coolant and the two cryostats are aligned so that the field coils have a common axis **12**. The support pillars **35a, 35b** and support tubes **17** of the spacer tube assembly **16** maintains the alignment of the magnetic field coils within each cryostat to lie along a common axis **12**. The length of the support tubes **17** of the spacer tube assembly **16** determines the spacing between the two cryostats **30a** and **30b**, and the length of the support pillars **35** determines the radial distance of open space **11**.

Each structural parts of the magnet including the toroidal chambers, the support pillars, and the support tube, all have thermal shields around them and are cooled to nearly the same low temperature. They are highly shielded from room temperature variations, which could cause uneven expansion of some of the parts leading to changes in magnetic field strength and uniformity. This is necessary to provide clear and accurate imaging data.

Additionally, two cryostats may be separately boxed and shipped to the final destination. Since each cryostat is roughly half the size and half the weight of the complete magnet, the parts are much easier to handle, enabling the use of lighter weight equipment to handle the shipment. Also the smaller size allows for easy entrance to facilities with narrow doorways or passageways. Each cryostat can be shipped on a separate pallet and then assembled with simple equipment. The final assembly at the final destination dose not requires any extra equipment other than a socket wrench, which is normally part of the standard final assembly equipment.

While several different features and embodiments of the invention have been described it will be clear that variations in the details of the embodiments specifically illustrated and describe may be made without departing from the true spirit of invention as defined in the appended claims.

## Claims

1. A superconducting magnet arrangement (10), the magnet arrangement comprising:
a pair of spaced apart coil members (34a, 34a', 34a"; 34b, 34b', 34b"), wherein the coil members are axially aligned along a common axis (12);
a pair of spaced apart preferably toroidal chambers (33a, 33b), wherein the chambers are aligned along the common axis (12), wherein each chamber houses one of the coil members (34a, 34a', 34a"; 34b, 34b', 34b") and wherein each chamber is adapted to receive and store a liquid coolant;
a plurality of support structures (17, 35a, 35b) arranged about a periphery of the chambers (33a, 33b) and mechanically coupled therewith providing a predetermined gap between said chambers and fixing the position of said superconducting coil members (34a, 34a', 34a"; 34b, 34b', 34b").

2. Arrangement of claim 1, wherein each said support structure (17, 35a, 35b) comprises:
a pair of support pillars (35a, 35b), each with a base end mechanically and thermally attached to an outer surface of a respective chamber (33a, 33b) and protruding therefrom to a far end being opposite to the base end, wherein in particular the support pillars are radially extending from the surface of the respective chambers.

3. Arrangement of claim 1 or 2, wherein each said support structure (17, 35a, 35b) comprises:
a support spacer (17) positioned parallel to the common axis (12) and mechanically and thermally coupled to the far ends of support pillars (35a, 35b), wherein in particular the support spacer is a support bar or a support tube.

4. Arrangement of any of the previous claims, further comprising:
a thermal shield (18, 32a, 32b) surrounding the plurality of support structures (17, 35a, 35b) and the pair of chambers (33a, 33b), and
an outer vacuum-tight case (19, 31 a, 31b) external to said thermal shield (18, 32a, 32b) providing thermal insulation.

5. Arrangement of any of the previous claims, further comprising a cryogenic cooler (39a, 39b), wherein in particular the cryogenic cooler is mounted on an outer surface of the vacuum-tight case (19, 31 a, 31b) and thermally coupled to the thermal shield (18, 32a, 32b).

6. Arrangement of claim 4 or 5, wherein a vacuum chamber is formed by the outer vacuum-tight case (19, 3 1 a, 31 b), wherein in particular the vacuum chamber is formed between the outer vacuum-tight case (19, 31 a, 31 b) and the outer surface of the chambers (33a, 33b) and within said support structure (17, 35a, 35b).

7. Arrangement according to any of the previous claims, wherein each of the support structures comprises a support spacer (17) axially extending preferably parallel to the common axis (12) and axially being enclosed by a thermal shield sleeve (18) and/or an outer vacuum cover sleeve (19), wherein in particular the axial length of the thermal shield sleeve (18) and/or the outer vacuum cover sleeve (19) is variable or adjustable before mounting.

8. A method of forming of a superconducting magnet arrangement (10), in particular a superconducting magnet arrangement according to any of the previous claims, the method comprising the steps of:
orienting and coupling two cryostats (30a, 30b), each with a chamber (33a, 33b), via two, three, four or more support structures (35a, 35b), each having a pair of support pillars (35a, 35b) and a support spacer (17);
positioning within each said chamber (33a, 33b) a superconducting coil member (34a, 34a', 34a"; 34b, 34b', 34b"), the coil members having a common axis (12);
placing a thermal shield (18, 32a, 32b) around each support structure (17, 35a, 35b) and each chamber (33a, 33b), wherein the thermal shield comprises a thermal shield sleeve (18) of adjustable length around the support spacer (17) of each support structure (17, 35a, 35b);
positioning a vacuum case (19, 31a, 31b) surrounding said thermal shield (18, 32a, 32b), wherein the vacuum case comprises a vacuum cover sleeve (19) of adjustable length around each one of the thermal shield sleeves (18);
coupling mechanically and thermally each of the support spacers (17) by coupling means to one of the pairs of the support pillars (35a, 35b), wherein the support pillars are mounted extending outwardly from an outer surface of the chambers (33a, 33b);
adjusting the length of the thermal shield sleeves (18) and coupling the thermal shield casings to the thermal shield arranged around the respective pair of support pillars (35a, 35b);
adjusting the length of the vacuum cover sleeve (19) and coupling the vacuum cover sleeves (19) to the vacuum case (19, 31 a, 31 b) arranged around the respective pair of support pillars (35a, 35b); and
feeding a liquid coolant via a coolant inlet port (38a, 38b) coupled to each one of the chambers (33a, 33b).

9. Method of claim 8, further comprising the steps of:
mounting each one of the support spacers (17) to a respective one of the support structures (17, 35a, 35b) of the first one of the cryostats (30a);
slipping each of the thermal shield sleeves (18) over a corresponding one of the attached support spacers (17) and bolting them to the first cryostat (30a);
providing O-ring seals (46) at both ends of the vacuum cover sleeves (19);
slipping one outer vacuum cover sleeve (19) over each one of the thermal shield sleeves (18) and bolting them to the first cryostat (30a);
shortening an effective length of both the vacuum cover sleeves (19) and the thermal shield sleeves (18) by pushing on the exposed ends thereby exposing protruding ends of the support spacers (17);
aligning the two cryostats (30a, 30b), and joining the protruding ends of the support spacers (17) to the second one of the cryostats (30b) by mounting the protruding end of each of the support spacers to a corresponding one of the cryostats support pillars (35a, 35b);
sliding the thermal shield sleeves (18) out to match with a corresponding one of connect sections arranged at the thermal shield (32b) of the second cryostat (30b) and joining together; and
sliding the outer vacuum cover sleeves (19) out to match with the corresponding one of connection sections arranged at the vacuum-tight case (31 b) of the second cryostat (30b) and joining together.

10. Method of claim 8 or 9, further comprising the step of shipping the two cryostats (30a, 30b) separately to the point of destination, in particular after the step of adjusting length of the thermal shield sleeves (18) and length of the cover sleeves (19).

11. Method of claim 8, 9 or 10, wherein the support spacer (17) is a support bar or a support tube, wherein in particular the method comprises:
before mounting the support spacers (17), aligning the longitudinal axis of the support spacer parallel to the common axis (12); and/or
wherein mounting the support spacers comprises mounting one or both axial ends of the support spacers to distal or outer ends of the support pillars (35a, 35b).

12. Arrangement of any of claims 1 to 7 or method according to any of claims 8 to 11, wherein the plurality of support structures (17, 35a, 35b) comprises two, three, four or more support structures arranged about the common axis (12) at equal angle or approximately equal angle intervals therebetween.

13. Arrangement according to claim 7 or 12 or the method of any of claims 8 to 12, wherein the outer vacuum cover sleeve (19) comprises a spacer cover flange (44) at one or at both axial ends, the spacer cover flange being adapted to be connected to a mating flange arranged at an outer vacuum-tight case (31 a, 31 b) of the support pillar (35a, 35b), and/or
wherein the thermal shield sleeve (18) comprises a shield flange (43) at one or at both axial ends, the shield flange being adapted to be connected to a mating flange arranged at a thermal shield (32a, 32b) of the support pillar (35a, 35b).

14. Arrangement according to any of claims 1 to 7, 12 or 13 or the method according to any of claims 8 to 13, wherein each superconducting coil member (34a, 34a', 34a"; 34b, 34b', 34b") comprises one, two, three, four or more spaced apart superconducting coil windings.

15. Modular superconducting magnet kit comprising:
a pair of coil members (34a, 34a', 34a"; 34b, 34b', 34b"), wherein each coil member comprises at least one coil winding made of superconducting material;
a pair of preferably toroidal chambers (33a, 33b) as a part of a cryostat (30a, 30b), wherein each chamber houses one of the coil members (34a, 34a', 34a"; 34b, 34b', 34b") and wherein each chamber is adapted to receive and store a liquid coolant;
a plurality of support structures (17, 35a, 35b) each comprising a pair of support pillars (35a, 35b) and a support spacer (17), and
for each support spacer (17) a thermal shield (18) at least partially surrounding the support spacer when mounted and a vacuum cover sleeves (19) at least partially surrounding the thermal shield (18) when mounted,
wherein the support pillars (35a, 35b) are mounted at or are adapted to be mounted at an outer surface of the chambers (33a, 33b), a first of each of the pair of pillars mounted or mountable to a first one of the chambers and a second of each of the pairs of pillars mounted or mountable to a second one of the chambers,
wherein when mounted, the support pillars protrude from the outer surface of the chambers and extend beyond the outer dimension of the chambers, and
wherein for each one of the support structures, a first end of the support spacer is connectable to a distal or outer end of the first one of the pair of support pillars and a second end of the support spacer is connectable to a distal or outer end of the second one of the pair of support pillars,
such that when the support pillars are mounted at the chambers and the support spacers are mounted between the corresponding ones of the pairs of support pillars, the superconducting coil members in the chambers are mounted parallel to each other having a common axis (12) of symmetry and a predetermined gap is provided between the respective vacuum casings (31a, 31b) of said chambers, wherein preferably the protruding extension of the support pillars is such that a clear space (11) is extending around the outer circumference of the gap such as to expand the clearance of the gap at least in radial direction.

## Amended claims

### Amended claims in accordance with Rule 137(2) EPC.

**1.** A superconducting magnet arrangement (10), the magnet arrangement comprising:
a pair of spaced apart coil members (34a, 34a', 34a"; 34b, 34b', 34b"), wherein the coil members are axially aligned along a common axis (12);
a pair of spaced apart preferably toroidal chambers (33a, 33b), wherein the chambers are aligned along the common axis (12), wherein each chamber houses one of the coil members (34a, 34a', 34a"; 34b, 34b', 34b") and wherein each chamber is adapted to receive and store a liquid coolant; and
a plurality of support structures (17, 35a, 35b) arranged about a periphery of the chambers (33a, 33b) and mechanically coupled therewith providing a predetermined gap between said chambers and fixing the position of said superconducting coil members (34a, 34a', 34a"; 34b, 34b', 34b");
wherein each said support structure (17, 35a, 35b) comprises:
a pair of support pillars (35a, 35b), each with a base end mechanically and thermally attached to an outer surface of a respective chamber (33a, 33b) and protruding therefrom to a far end being opposite to the base end, and
a support spacer (17) extending preferably parallel to the common axis (12) and mechanically and thermally coupled to the far ends of support pillars (35a, 35b);
**characterized by**
a thermal shield (18, 32a, 32b) surrounding the plurality of support structures (17, 35a, 35b) and the pair of chambers (33a, 33b), and
an outer vacuum-tight case (19, 31a, 31b) external to said thermal shield (18, 32a, 32b) providing thermal insulation.

**2.** Arrangement of claim 1, wherein the support pillars (35a, 35b) are radially extending from the surface of the respective chambers (33a, 33b).

**3.** Arrangement of claim 1 or 2, wherein the support spacer (17) is a support bar or a support tube.

**4.** Arrangement of any of the previous claims, further comprising a cryogenic cooler (39a, 39b), wherein in particular the cryogenic cooler is mounted on an outer surface of the vacuum-tight case (19, 31a, 31b) and thermally coupled to the thermal shield (18, 32a, 32b).

**5.** Arrangement of any of the previous claims, wherein a vacuum chamber is formed between the outer vacuum-tight case (19, 31a, 31b) and the outer surface of the chambers (33a, 33b) and within said plurality of support structures (17, 35a, 35b).

**6.** Arrangement according to any of the previous claims,
wherein said thermal shield (18, 32a, 32b) comprises thermal shield sleeves (18), wherein said outer vacuum-tight case (19, 31a, 31b) comprises vacuum cover sleeves (19), and
wherein each of the support spacers (17) is axially enclosed by one of said thermal shield sleeves (18) and one of said vacuum cover sleeves (19), wherein in particular the axial length of the thermal shield sleeves (18) and/or the vacuum cover sleeves (19) is variable or adjustable before mounting.

**7.** A method of forming of a superconducting magnet arrangement (10), in particular a superconducting magnet arrangement according to any of the previous claims, the method comprising the steps of:
orienting and coupling two cryostats (30a, 30b), each with a chamber (33a, 33b), via two, three, four or more support structures (35a, 35b), each having a pair of support pillars (35a, 35b) and a support spacer (17), arranged about a periphery of the chambers (33a, 33b) and providing a predetermined gap between said chambers;
positioning within each said chamber (33a, 33b) a superconducting coil members (34a, 34a', 34a"; 34b, 34b', 34b"), the coil members having a common axis (12);
placing a thermal shield (18, 32a, 32b) around each support structure (17, 35a, 35b) and each chamber (33a, 33b), wherein the thermal shield comprises a thermal shield sleeve (18) of adjustable length around the support spacer (17) of each support structure (17, 35a, 35b);
positioning a vacuum case (19, 31a, 31b) surrounding said thermal shield (18, 32a, 32b), wherein the vacuum case comprises a vacuum cover sleeve (19) of adjustable length around each one of the thermal shield sleeves (18);
coupling mechanically and thermally each of the support spacers (17) by coupling means to one of the pairs of the support pillars (35a, 35b), wherein the support pillars are mounted extending outwardly from an outer surface of the chambers (33a, 33b);
adjusting the length of the thermal shield sleeves (18);
adjusting the length of the vacuum cover sleeves (19) and
feeding a liquid coolant via a coolant inlet port (38a, 3 8b) coupled to each one of the chambers (33a, 33b).

**8.** Method of claim 7, wherein the steps placing a thermal shield, positioning a vacuum case and coupling mechanically, thermally each of the support spacers, adjusting the length of the thermal shield sleeves, and adjusting the length of the vacuum cover sleeves include in this order the following partial sub-steps:
mounting each one of the support spacers (17) to a respective one of the support structures (17, 35a, 35b) of the first one of the cryostats (30a);
slipping each of the thermal shield sleeves (18) over a corresponding one of the attached support spacers (17) and bolting them to the first cryostat (30a);
providing O-ring seals (46) at both ends of the vacuum cover sleeves (19);
slipping one vacuum cover sleeve (19) over each one of the thermal shield sleeves (18) and bolting them to the first cryostat (30a);
shortening an effective length of both the vacuum cover sleeves (19) and the thermal shield sleeves (18) by pushing on the exposed ends thereby exposing protruding ends of the support spacers (17);
aligning the two cryostats (30a, 30b), and joining the protruding ends of the support spacers (17) to the second one of the cryostats (30b) by mounting the protruding end of each of the support spacers to a corresponding one of the cryostats support pillars (35a, 35b);
sliding the thermal shield sleeves (18) out to match with a corresponding one of connect sections arranged at the thermal shield (32b) of the second cryostat (30b) and joining together; and
sliding the vacuum cover sleeves (19) out to match with the corresponding one of connection sections arranged at the vacuum-tight case (31b) of the second cryostat (30b) and joining together.

**9.** Method of claim 7, wherein before the step of coupling mechanically and thermally each of the support spacers (17) the method further comprises the step of shipping the two cryostats (30a, 30b) separately to the point of destination.

**10.** Method of claim 7, 8 or 9, wherein the support spacer (17) is a support bar or a support tube, wherein in particular the method comprises:
before mounting the support spacers (17), aligning the longitudinal axis of the support spacer parallel to the common axis (12); and/or
wherein mounting the support spacers comprises mounting one or both axial ends of the support spacers to distal or outer ends of the support pillars (35a, 35b).

**11.** Arrangement of any of claims 1 to 6 or method according to any of claims 7 to 10, wherein the plurality of support structures (17, 35a, 35b) comprises two, three, four or more support structures arranged about the common axis (12) at equal angle or approximately equal angle intervals therebetween.

**12.** Arrangement according to any of claims 1 to 6 or 11 or the method of any of claims 7 to 11,
wherein the outer vacuum-tight case (19, 31a, 31b) comprises for each of the chambers (33a, 33b) a vacuum case (31a, 31b) each surrounding a respective one of the chambers and the support pillars (35a, 35b) attached thereto and
wherein the vacuum cover sleeve (19) comprises a spacer cover flange (44) at one or at both axial ends, the spacer cover flange being adapted to be connected to a mating flange arranged at the vacuum case (31a, 31b), and/or
wherein the thermal shield sleeve (18) comprises a shield flange (43) at one or at both axial ends, the shield flange being adapted to be connected to a mating flange arranged at a thermal shield (32a, 32b) of the support pillar (35a, 35b).

**13.** Arrangement according to any of claims 1 to 6, 11 or 12 or the method according to any of claims 7 to 12, wherein each superconducting coil member (34a, 34a', 34a"; 34b, 34b', 34b") comprises one, two, three, four or more spaced apart superconducting coil windings.

**14.** Modular superconducting magnet kit comprising:
a pair of coil members (34a, 34a', 34a"; 34b, 34b', 34b"), wherein each coil member comprises at least one coil winding made of superconducting material;
a pair of preferably toroidal chambers (33a, 33b) as a part of a cryostat (30a, 30b), wherein each chamber houses one of the coil members (34a, 34a', 34a"; 34b, 34b', 34b") and wherein each chamber is adapted to receive and store a liquid coolant; and
a plurality of support structures (17, 35a, 35b) each comprising a pair of support pillars (35a, 35b) and a support spacer (17), which, when mounted, are arranged about a periphery of the chambers (33a, 33b) and provide a predetermined gap between said chambers,
wherein the support pillars (35a, 35b) are adapted to be mounted at an outer surface of the chambers (33a, 33b), a first of each of the pair of pillars adapted to be mounted to a first one of the chambers and a second of each of the pairs of pillars adapted to be mounted to a second one of the chambers,
wherein when mounted, the support pillars protrude from the outer surface of the chambers and extend beyond the outer dimension of the chambers, and
**characterized by**
, for each support spacer (17), a thermal shield (18) at least partially surrounding the support spacer when mounted and a vacuum cover sleeves (19) at least partially surrounding the thermal shield (18) when mounted,
wherein for each one of the support structures, a first end of the support spacer is adapted to be connected to a distal or outer end of the first one of the pair of support pillars and a second end of the support spacer is adapted to be connected to a distal or outer end of the second one of the pair of support pillars,
such that when the support pillars are mounted at the chambers and the support spacers are mounted between the corresponding ones of the pairs of support pillars, the superconducting coil members in the chambers are mounted parallel to each other having a common axis (12) of symmetry and a predetermined gap is provided between the respective vacuum casings (31a, 31b) of said chambers,
wherein preferably the extension of the support pillars beyond the outer dimension of the chambers is such that a clear space (11) is extending around the outer circumference of the gap such as to expand the clearance of the gap at least in radial direction.
